# EUROPEAN PATENT APPLICATION

(11) **EP 4 282 871 A1**
(43) Date of publication of application: **29.11.2023**
(21) Application number: 21920501.0
(22) Date of filing: 02.06.2021
(51) Int. Cl.: C07H 21/04, C07H 1/00, C12P 19/34

(54) **METHOD FOR MODIFYING DNA WITH GLYCOSIDASE AND OXYLAMINE COMPOUND**

(30) Priority: 22.01.2021 CN 202110088599
(71) Applicant: Nanjing University, Nanjing, Jiangsu 210023 (CN)
(72) Inventor: YU, Hanyang, Nanjing, Jiangsu 210023 (CN); YANG, Jintao, Nanjing, Jiangsu 210023 (CN); LI, Zhe, Nanjing, Jiangsu 210023 (CN)
(74) Representative: Murgitroyd & Company
(86) International application number: PCT/CN2021/097832
(87) International publication number: WO 2022/156117

(57) **Abstract**

The present invention discloses a method for modifying a DNA by utilizing a glycosidase and an oxyamine compound, including the following steps: conducting solid phase synthesis of a DNA strand carrying an atypical base; reacting the DNA strand carrying an atypical base under the catalysis of the glycosidase which selectively recognizes the atypical base, to produce a DNA strand carrying a base-deficient site; and reacting the DNA strand carrying the base-deficient site with the oxyamine compound to generate a DNA modified with a chemical functional group. This method can realize diversified site-directed modification of the DNA, study the base structure-function relationship of a functional DNA, and construct a functional DNA with stronger activity. The present method is simple and easy to use, and can reduce the cost of modification and the time required for modification. The modified functional DNA has higher activity and can provide better tool molecules for biotechnology and disease diagnosis and treatment.

## Description

### TECHNICAL FIELD

The present disclosure relates to a method for modifying a DNA by utilizing a glycosylase and an oxyamine compound, and in particular to a method for modifying a functional DNA by utilizing a glycosylase and an oxyamine compound.

### BACKGROUND

A functional DNA is a DNA with binding ability or catalytic activity, which is obtained through in vitro selection and folds to form a stable three-dimensional structure. Generally, functional DNAs include two categories: DNA aptamers and deoxyribozymes. The DNA aptamer can bind to a specific target, for example a protein, a small molecule and the like, and can be used for target detection and function regulation, etc. The deoxyribozyme can catalyze various chemical reactions, and can be applied in compound preparation, molecular detection and gene silencing, etc. The functional DNA has a wide range of application, such as disease diagnosis and treatment.

Although functional DNAs have many advantages and applications, the functions and applications of functional DNAs are still limited. One reason is that the nucleobases of natural DNAs have fewer functional groups compared to the side chains of amino acids, the basic building blocks of proteins. This problem can be solved by chemical modification of the DNA nucleobases to introduce more diverse chemical functional groups. An existing DNA modification method mainly uses means of synthetic chemistry to prepare monomers containing bases modified with various kinds of functional groups, and uses these DNA monomers containing modified bases in a solid phase synthesis process to finally produce a DNA strand containing a site-specific chemical modification. This method can introduce more functional groups into the DNA, but there are many steps in the synthesis method, and the required reaction conditions are relatively harsh, which limits the types of functional groups that can be synthesized, while the required material cost and time cost is high.

### SUMMARY

Objective of the present invention: an objective of the present disclosure is to provide a simple and easily accessible modification method with low cost and short time consumption for DNA modification.

Technical solution: a method for modifying a DNA with a glycosylase and an oxyamine compound according to the present invention, which includes the following steps:
(1) conducting solid phase synthesis of a DNA strand carrying a non-canonical base;
(2) reacting the DNA strand carrying a non-canonical base under the catalysis of the glycosylase which selectively recognizes the non-canonical base, to produce a DNA strand carrying an abasic site; and
(3) reacting the DNA strand carrying the abasic site with the oxyamine compound to generate a DNA modified with a chemical functional group.

The functional DNA modified by the specific oxyamine compound at a specific site can have better functionality.

Meanwhile, removal of various non-canonical bases and modification with various oxyamine compounds can be realized through orthogonal base-glycosylase pairs, achieving modification with various different oxyamine compounds at multiple sites of the same DNA strand.

Because the glycosylase that can be used in the present method can be a commercially available uracil DNA glycosylase, etc., and a commercially available common oxyamine compound can also be used as the oxyamine compound, the raw materials are easy to obtain.

The abasic site in the step (2) is a sugar ring without a base after treatment with the glycosylase. The incubation and separation time in the step (2) can be realized within 2 hours, and the reaction between the oxyamine compound and the DNA containing the abasic site in the step (3) is rapid and can be completed within half an hour, so the whole modification process can be completed quickly.

Different glycosylases specifically remove corresponding non-canonical bases in the DNA to obtain abasic sites. Multiple identical functional groups can be simultaneously introduced into multiple sites for modification by introducing multiple identical non-canonical bases into the same DNA strand. Multiple different non-canonical bases are introduced into the same DNA strand, and then the DNA strand is treated by using corresponding glycosylases and oxyamine compounds sequentially to obtain a DNA containing different modifications at different sites.

The non-canonical base in the step (1) includes one or more of the following: uracil, hypoxanthine, 3-methyladenine, 3-methylguanine, 7-methylguanine or oxoguanine, etc., and the glycosylase in the step (2) includes one or more of the following: a uracil DNA glycosylase, a methylated purine DNA glycosylase or a 8-oxoguanine DNA glycosylase, etc.

The step (1) specifically includes: designing a specific modification site and selecting a corresponding non-canonical base, and synthesizing a corresponding DNA single strand and a corresponding complementary DNA single strand.

The step (2) specifically includes: incubating the single strand synthesized in the step (1) with the corresponding glycosylase in a corresponding buffer for a certain period of time, and then separating the single strand from the glycosylase by an ethanol precipitation method. Alternatively, the single strand synthesized in the step (1) is annealed to a complementary strand carrying a biotin label to obtain a double strand, incubated with the corresponding glycosylase in the corresponding buffer for a certain time, and then the double strand is separated from the glycosylase by the ethanol precipitation method.

The step (3) specifically includes: incubating the DNA single strand or double strand carrying an abasic site obtained in the step (2) with an oxyamine compound in a buffer for a certain period of time. After the reaction, a modified DNA single strand is obtained by desalting the single strand directly with a desalting column, or alternatively a modified DNA single strand is obtained by using a streptavidin separation method on the modified DNA double strand.

Beneficial effect: compared with the prior art, the present disclosure has the following obvious advantages:
(1) the modification method is simple and easy to conduct;
(2) the compound limitation in chemical synthesis is avoided, and a number of functional groups can be selected;
(3) the modification cost is low; and
(4) the time required for modification is short.

### BRIEF DESCRIPTION OF DRAWINGS

Fig. 1 is a schematic diagram of modifying a DNA with methoxyamine by using a uracil DNA glycosylase;
Fig. 2 shows modification sites and cleavage rate characterization for a modified deoxyribozyme 10-23;
Fig. 3 shows an example of an oxyamine compound as used; and
Fig. 4 is a schematic diagram of conducting two-site site-directed modification.

### DETAILED DESCRIPTION

The technical solution of the present disclosure will be further explained in connection with specific examples hereafter.

### Example 1

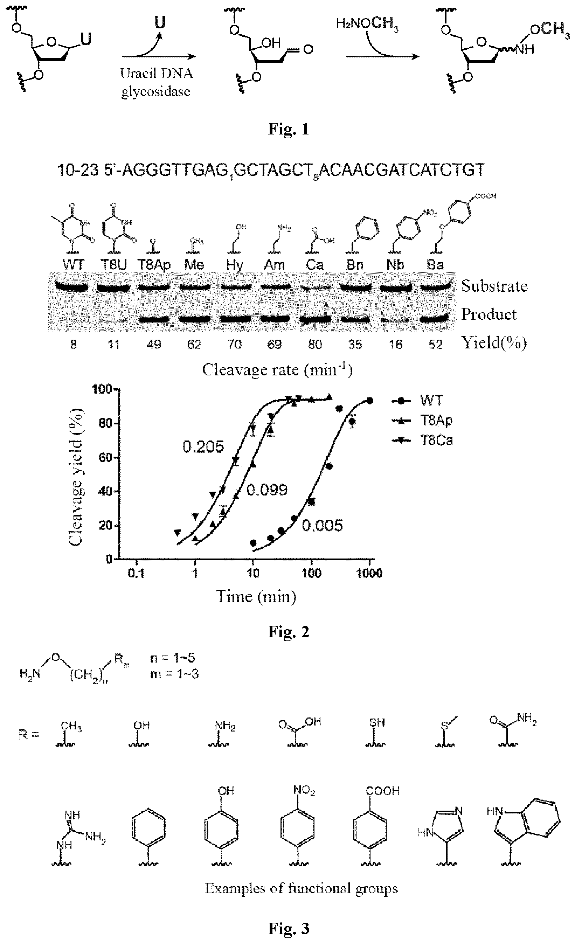

As shown in Fig. 1, a DNA single strand containing a uracil base was synthesized, and then uracil was removed by using a uracil DNA glycosylase. The reaction conditions were 10 µM DNA, 50 U/ml of the uracil DNA glycosylase (UDG), 20 mM Tris-HCl, 1 mM DTT, 1 mM EDTA, pH 8 (@ 25°C) and incubation at 37°C for 30 minutes. After the reaction, a DNA carrying an abasic site was purified by ethanol precipitation, then added with methoxyamine under conditions of 5 mM methoxyamine, 50 mM HEPES, 100 mM NaCl, pH 7.0, and incubated at 37°C for 30 minutes, so as to generate a DNA single strand modified with methyl through reaction. The DNA modified with methyl was purified by ethanol precipitation. The total time of the whole modification process was 2.5 hours.

### Example 2

As shown in Fig. 2, a deoxyribozyme 10-23 was modified at a base T at position 8 of a catalytic loop. From left to right, 10 sequences were respectively wild-type 10-23 (WT), modified deoxyribozyme 10-23 with a uracil at position 8 (T8U), with an abasic site at position 8 (T8Ap), methyl at position 8 (Me), hydroxyethyl at position 8 (Hy), aminoethyl at position 8 (Am), carboxymethyl at position 8 (Ca), benzyl at position 8 (Bn), p-nitrobenzyl at position 8 (Nb), and carboxybenzyl at position 8 (Ba). The modified deoxyribozyme 10-23 was used in a substrate RNA cleavage experiment, and the cleaved products were verified by denaturing polyacrylamide gel electrophoresis. Under reaction conditions of 1 mM magnesium ions, 20 mM Tris-HCl, pH 7.5 and a cleavage time of 10 minutes, it could be seen that the cleavage yield of the modified deoxyribozyme on the substrate was improved to different degrees, indicating that the enzyme activity was improved. Furthermore, the cleavage yields of the wild-type 10-23 (WT), the deoxyribozyme 10-23 with an abasic site at position 8 and the modified deoxyribozyme 10-23 with a carboxyethyl group at position 8 were characterized over time, and the cleavage rates were calculated. It could be obtained that the cleavage rate of the deoxyribozyme 10-23 modified with carboxyethyl at position 8 was about 40 times higher than that of the wild-type.

### Example 3

As shown in Fig. 3, a functional group of an oxyamine compound that could be used might be methyl, hydroxyl, amino, carboxyl, thiol, methylthiol, amide, guanidino, phenyl, hydroxyphenyl, nitrophenyl, carboxyphenyl, imidazolyl and indolyl, and the linker could be a carbon chain isomer with 1 to 5 carbon atoms, and the number of functional groups was 1 to 3. The aforementioned oxyamine compound was reacted with a DNA carrying an abasic site under conditions of 5 mM of the oxyamine compound, 50 mM HEPES, 100 mM NaCl, pH 7.0, incubated at 37°C for 30 minutes, and then purified by ethanol precipitation.

### Example 4

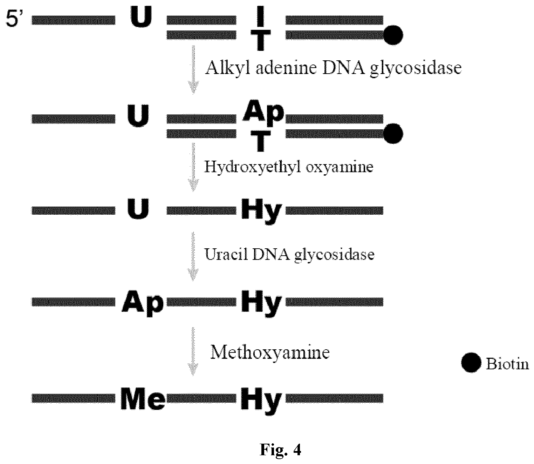

As shown in Fig. 4, a DNA single strand carrying a uracil (U) and a hypoxanthine (I) was synthesized, and the DNA single strand was annealed to a complementary strand modified with biotin under conditions of 85°C for 5 minutes and 4°C for 5 minutes, and then the hypoxanthine was removed by using an alkyl adenine DNA glycosylase under reaction conditions of 2 µM DNA, 200 U/ml of the alkyl adenine DNA glycosylase, 50 mM NaOAc, 1 mM DTT, 1 mM EDTA, 100 mM NaCl, 0.1 mg/ml BSA, pH 5.8, incubated at 37°C for 4 hours, then purified by ethanol precipitation to obtain a DNA carrying an abasic site. The DNA carrying an abasic site was reacted with hydroxyethoxyamine under conditions of 5 mM of the hydroxyethoxyamine, 50 mM HEPES, 100 mM NaCl, pH 7.0, incubated at 37°C for 30 minutes, and then separated by using a streptavidin gel to obtain a modified single strand, i.e. a DNA single strand modified with uracil and hydroxyethyl. Then, the uracil was removed by using a uracil DNA glycosylase under reaction conditions of 10 µM DNA, 50 U/ml of the uracil DNA glycosylase (UDG), 20 mM Tris-HCl, 1 mM DTT, 1 mM EDTA, pH 8 (@25°C) and incubating at 37°C for 30 minutes. After the reaction, a DNA carrying an abasic site was purified by ethanol precipitation, then added with methoxyamine under conditions of 5 mM of the methoxyamine, 50 mM HEPES, 100 mM NaCl, pH 7.0, and incubated at 37°C for 30 minutes, so as to generate a DNA single strand modified with methyl through the reaction. The DNA single strand modified with methyl was purified by ethanol precipitation. The time of the whole modification process was 8.5 hours.

## Claims

1. A method for modifying a DNA by utilizing a glycosylase and an oxyamine compound, comprising the following steps:
(1) conducting solid phase synthesis of a DNA strand carrying a non-canonical base;
(2) reacting the DNA strand carrying a non-canonical base under the catalysis of the glycosylase which selectively recognizes the non-canonical base, to produce a DNA strand carrying an abasic site; and
(3) reacting the DNA strand carrying the abasic site with the oxyamine compound to generate a DNA modified with a chemical functional group.

2. The method for modifying a DNA by utilizing a glycosylase and an oxyamine compound according to claim 1, wherein the abasic site in the step (2) is a sugar ring site without a base after treatment with the glycosylase.

3. The method for modifying a DNA by utilizing a glycosylase and an oxyamine compound according to claim 1, wherein the oxyamine compound in the step (3) is an oxyamine compound having one or more of the following methyl, hydroxyethyl, aminoethyl, carboxymethyl or benzyl.

4. The method for modifying a DNA by utilizing a glycosylase and an oxyamine compound according to claim 1, wherein different glycosylases specifically remove corresponding non-canonical bases in the DNA to obtain abasic sites.

5. The method for modifying a DNA by utilizing a glycosylase and an oxyamine compound according to claim 1, wherein multiple identical functional groups are simultaneously introduced into multiple sites for modification by introducing multiple identical non-canonical bases into the same DNA strand.

6. The method for modifying a DNA by utilizing a glycosylase and an oxyamine compound according to claim 1, wherein multiple different non-canonical bases are introduced into the same DNA strand, and then the DNA strand is treated by using multiple glycosylases and oxyamine compounds sequentially to obtain a DNA containing different modifications at different sites.

7. The method for modifying a DNA by utilizing a glycosylase and an oxyamine compound according to claim 1, wherein the non-canonical base is one or more of the following: uracil, hypoxanthine, 3-methyladenine, 3-methylguanine, 7-methylguanine or oxoguanine.

8. The method for modifying a DNA by utilizing a glycosylase and an oxyamine compound according to claim 1, wherein the glycosylase is one or more of the following: a uracil DNA glycosylase, a methylated purine DNA glycosylase or an 8-oxoguanine DNA glycosylase.

9. The method for modifying a DNA by utilizing a glycosylase and an oxyamine compound according to claim 1, wherein the step (1) specifically comprises the following steps: designing a specific modification site and selecting a corresponding non-canonical base, and synthesizing a corresponding DNA single strand and a corresponding complementary DNA single strand.

10. The method for modifying a DNA by utilizing a glycosylase and an oxyamine compound according to claim 1, wherein the step (2) specifically comprises the following steps: incubating the DNA single strand carrying the non-canonical base with the corresponding glycosylase in a buffer, and then separating the single strand from the glycosylase by using an ethanol precipitation method; or alternatively annealing the DNA single strand carrying the non-canonical base to a complementary strand carrying a biotin label to obtain a double strand, adding the corresponding glycosylase and incubating in a buffer, and then separating the double strand from the glycosylase by using the ethanol precipitation method.

11. The method for modifying a DNA by utilizing a glycosylase and an oxyamine compound according to claim 1, wherein the step (3) specifically comprises the following steps: incubating the DNA single strand or double strand carrying the abasic site with the oxyamine compound in a buffer; and after the reaction, desalting the single strand with a desalting column to obtain a modified DNA single strand, or alternatively obtaining a modified DNA single strand by using a streptavidin separation method on the modified DNA double strand.
